# EUROPÄISCHE PATENTSCHRIFT

(11) **EP 1 177 773 B1**
(45) Veröffentlichungstag und Bekanntmachung des Hinweises auf die Patenterteilung: **04.06.2003**
(21) Anmeldenummer: 01118312.6
(22) Anmeldetag: 27.07.2001
(51) Int. Cl.: A61B 19/00

(54) **Verfahren und Vorrichtung zur Bestimmung und Abgleichung der Raumkoordinaten eines chirurgischen Instrumentes**
Method and device for determination and calibration of coordinates of a surgical instrument
Procédé et appareil pour la détermination et la calibration des coordonnées d'un instrument chirurgical

(30) Priorität: 03.08.2000 DE 10037840
(43) Veröffentlichungstag der Anmeldung: 06.02.2002
(73) Patentinhaber: Drosten, Robert, 78713 Schramberg (DE)
(72) Erfinder: Drosten, Robert, 78713 Schramberg (DE)
(74) Vertreter: Engelhardt, Volker

(56) Entgegenhaltungen:
- EP-A- 0 868 886
- WO-A-98/15234
- DE-A- 19 902 273
- US-A- 6 006 126

## Beschreibung

Die Erfindung bezieht sich auf ein Verfahren zur Bestimmung und Abgleichung der Raumkoordinaten eines chirurgischen Instrumentes während einer dentalen Operation am Ober- und/oder Unterkiefer eines Patienten, sowie auf eine Vorrichtung zur Anwendung dieses Verfahrens nach den Oberbegriffen der Patentansprüche 1 und 7.

Aus der EP 97 116 843 A1 oder der EP 0 868 886 A1 ist ein Verfahren sowie eine Vorrichtung bekannt, mittels denen das zu operierende Körperteil und das chirurgische Instrument im Raum einem vorgegebenen Koordinatensystem zugeordnet werden, so dass die Position des chirurgischen Instrumentes in Abhängigkeit von erstellten Schichtbildaufnahmen des zu operierenden Körperteils ermittelbar ist.

Die Vorrichtung gemäß der EP 97 116 843 A1 umfasst dabei eine in den Mund des Patienten einzuführende Positionsplatte, die beispielsweise an einzelnen Zähnen, befestigt wird. An der Positionsplatte sind seitlich abstehende Sensoren tragende Kontrollptatten angebracht. Aufgrund der vorgegebenen und bekannten geometrischen Abmessungen dieser Positions- und Kontrollplatten kann über die Sensoren eine Zuordnung der Kieferposition des Patienten in bezug auf das Kartesische- Koordinatensystem erfolgen.

Eine optische Lichtquelle strahlt demzufolge die Sensoren an, so dass mittels der Reflexion des Lichtstrahles Rückschlüsse auf die Kieferposition möglich sind. Dadurch, dass die Vorrichtung auch beim Erstellen der Schichtbildaufnahmen im Kemspintomographen bereits in der entsprechenden Position angebracht ist, kann eine Transformationsmatrix zwischen dem Koordinatensystem des Kernspintomographen und dem Koordinatensystem des Operationsbereiches aufgestellt werden, so das ein Abgleich zwischen den tatsächlichen Koordinaten des chirurgischen Instrumentes mit den Koordinaten der erstellten Schichtbildaufnahmen möglich ist.

Als nachteilig bei dem bekannten Verfahren hat es sich gezeigt, dass in den Mund des Patienten bereits vor der Operation die unhandliche und schwere Vorrichtung zur Bestimmung der Kieferposition im Raum einzusetzen und sowohl die Schichtbildaufnahmen des Kiefers als auch die Operation mit eingesetzter Vorrichtung durchzuführen sind. Sobald jedoch die Vorrichtung zwischen den einzelnen Verfahrensabschnitten bzw. -schritten verrutscht oder gelockert wird, sind die bereits bestimmten Messergebnisse unbrauchbar und die Erstellung der Schichtbildaufnahmen hat von vorne zu beginnen.

Des weiteren stört die Vorrichtung während der Operation, da diese den Zugang zu dem zu operierenden Ober- oder Unterkiefer behindert.

Darüber hinaus sind zwei räumlich getrennte Koordinatensysteme zur Erfassung der jeweiligen Position der Vorrichtung notwendig, da zum einen die Schichtbildaufnahmen und die damit verbundene Position und Orientierung der Vorrichtung in dem Kemspintomographen und zum anderen die Positionen der Vorrichtung während der Operation zu bestimmen sind. Zusätzlich ist auch die Position und Orientierung des chirurgischen Instrumentes zu erfassen. Zwischen diesen ermittelten Raummatrixen, die auf unterschiedlichen Koordinatensystemen basieren, ist demnach ein mathematisch aufwendiger Abgleichungsprozess vorzunehmen.

Bei solchen Berechnungen sind Fehlabweichungen jedoch unvermeidlich, so dass der während der Operation vorgesehene Abgleich zwischen der vorgegebenen Sollposition und der tatsächlichen Istposition des chirurgischen Instrumentes bezogen auf den gewünschten Bohrkanal erhebliche Fehlabweichungen aufweist. Folglich kann die Bewegung des Instrumentes nicht zuverlässig überwacht und gegebenenfalls korrigiert werden.

In der DE 42 25 112 C1 ist eine Einrichtung zum Messen der Position eines Instrumentes relativ zu einem Behandlungsobjekt offenbart. Hierbei wird die Position und Ausrichtung des Operationsinstrumentes mittels einer an diesem befestigten Trägersensoreinheit bewerkstelligt, dessen Raumkoordinaten über eine an der Decke des Operationssaals angebrachten Messvorrichtung durchgeführt wird. Somit erfolgt die Positionsüberwachung des chirurgischen Instrumentes in einem Koordinatensystem, das mit dem Koordinatensystem der Schichtbildaufnahmen nicht korrespondiert..
Auch für diese Messeinrichtung ergeben sich daher die bereits angesprochenen Nachteile.

Es ist daher Aufgabe der Erfindung, ein Verfahren und zur Anwendung des Verfahrens eine Vorrichtung der eingangs genannten Gattung zu schaffen, mit deren Hilfe eine exakte Positionsbestimmung des chirurgischen Instrumentes während der Operation in Abhängigkeit von dem festgelegten Bohrkanal gewährleistet ist, ohne dass aufwendige und komplizierte mathematische Abgleichungsberechnungen durchzuführen sind.

Diese Aufgabe wird erfindungsgemäß durch die Verfahrensschritte gemäß des kennzeichnenden Teils des Patentanspruches 1 gelöst.

Zur optimalen Festlegung des Bohrkanals und zur Vermeidung von Fehlabweichungen wird während der Erstellung der Schichtbilder des zu operierenden Kiefers die Positionsabweichung des Kiefers für jedes Schichtbild an Hand der Positionsbestimmung der Referenzpunkte ermittelt.

Dadurch, dass sich der Ober- oder Unterkiefer während der Erstellung der einzelnen Schichtbildaufnahmen unweigerlich bewegt, sind die jeweiligen Schichtbildaufnahmen unter Berücksichtigung dieser Abweichungen mit einer Transformationsmatrix zu versehen, so dass durch die ermittelten Positionsabweichungen der Referenzpunkte eine Transformationsmatrix errechnet wird, mittels der die Schichtbilder in Abhängigkeit von einem vorgegebenen Sollwert kalibriert werden.

Nach der Ermittlung der Transformationsmatrix kann der optimale Bohrkanal festgelegt und die Operation durchgeführt werden. Zu diesem Zweck ist der Patient in den Operationssaal zu überführen. Mit Hilfe der Transformationsmatrix kann nunmehr jeder Schichtbildaufnahme innerhalb des Operationssaales eine Positionsabweichung des zu operierenden Ober- oder Unterkiefers zugeordnet werden, und zwar bezogen auf den vorgegebenen Sollwert des Bohrkanals.

Dadurch, dass der Ursprung des Kartesischen - Koordinatensystem, in dem die Raumkoordinaten in Position und Orientierung des chirurgischen Instrumentes erfasst werden, einem Referenzpunkt entspricht, ist gewährleistet, dass das Koordinatensystem der Schichtbildaufnahmen und das Koordinatensystem, in dem das chirurgische Instrument erfasst ist, identisch sind, so dass demzufolge mathematisch aufwendige Umrechnungen entfallen.

Für die Anwendung des Verfahrens wird eine Vorrichtung vorgeschlagen, mittels der das Instrument mit der Rechen- und Auswerteeinheit über mindestens ein Koordinatenmeßsystem verbunden ist und mittels der die Datenverarbeitungseinrichtung und mindestens ein Referenzpunkt am Ober- und/oder Unterkiefer des Patienten während der Erstellung der Schichtbildaufnahmen mit dem Koordinatenmeßsystem sowie der Rechen- und Auswerteeinheit gekoppelt sind.

Als bevorzugte Ausführungsform hat es sich gezeigt, wenn das Koordinatenmeßsystem aus mindestens sechs einzelnen mit Lichtstrahlen durchleuchteten Glasfaserkabeln gebildet ist und wenn die Biegung,. Reflexion, Brechung und Absorption der Glasfaserkabel die Berechnungsgrößen darstellen, mittels denen die Rechen- und Auswerteeinheit die Raumkoordinaten des am Instrument befestigten Endes der Glasfaserkabel errechnet.

Weitere vorteilhafte Weiterbildungen der Erfindung ergeben sich aus den Unteransprüchen.

Dadurch, dass mindestens ein Referenzpunkt im Bereich der durchzuführenden Operationsstelle festgelegt ist, die im übrigen an beliebig markanten Positionen des Kopfes vorgegeben sein können, beispielsweise an einem Zahn im Ober- und/ oder Unterkiefer, ist eine exakte Zuordnung der Raumposition des zu operierenden Oberoder Unterkiefers in Abhängigkeit von den erstellten Schichtbildaufnahmen möglich, so dass die Schichtbildaufnahmen jeweils einer exakten Raumposition zugeordnet sind, ohne dass der Patient Schmerzen ausgesetzt ist, aufgrund von aufgeschraubten Kopf-Halteringen und/oder in den Mund eingesetzten Reflexionselementen.

Die Messungen der Positionen des Ober- oder Unterkiefers sowie des Instrumentes erfolgt in einem einheitlichen Kartesischen - Koordinatensystem, so dass die derart ermittelten Positionswerte unmittelbar miteinander verglichen werden können und aufwendige Umrechnungsmatrixen nicht notwendig sind. Dies spart zum einen für die Operation erhebliche Zeit und zum anderen entstehen keine durch die Umrechnung und Messung begründeten fehlerhaften Abweichungen, so dass ein exakter Abgleich zwischen den jeweiligen Positionswerten durchgeführt werden kann.

Auch die aufgrund der möglichen Patientenbewegung während der Erstellung der Schichtbildaufnahmen und während der Operation entstehenden Messabweichungen werden erfasst und ausgewertet, so dass diese fehlerhaften Abweichungen bei dem Abgleich der Positionswerte bereits berücksichtigt sind, denn jedem Schichtbild wird eine Transformationsmatrix zugeordnet, die ausgehend von einer Sollposition des Patienten die tatsächliche Istposition während der Erstellung der Schichtbildaufnahmen und der eigentlichen Operation beinhaltet und somit an jede Schichtbildaufnahme zu der Sollposition kalibriert werden. Gleiches erfolgt während der Operation mit der Position des Patienten; so dass die aufgrund möglicher Bewegungen entstehenden Abweichungen ausreichend berücksichtigt sind, so dass die Spitze des Instrumentes tatsächlich an der Position angesetzt und geführt werden kann, die vorab durch den Operateur festgelegt wurde.

In der Zeichnung ist ein erfindungsgemäßes Ausführungsbeispiel dargestellt, das nachfolgend näher erläutert wird. Im einzelnen zeigt:
- Figur 1: einen Patienten während der Erstellung von Schichtbildaufnahmen des zu operierenden Ober- oder Unterkiefers, in perspektivischer Ansicht,
- Figur 2: den Kieferbereich des Patienten gemäß Figur 1 in vergrößerter Darstellung,
- Figur 3: den Unterkiefer des Patienten gemäß Figur 1, während der dentalen Operation, in Draufsicht, und
- Figur 4: die erstellten Schichtbildaufnahmen gemäß Figur 1 mit einem festgelegten Bohrkanal sowie die Durchführung der Operation am Unterkiefer des Patienten gemäß Figur 3, in Seitenansicht.

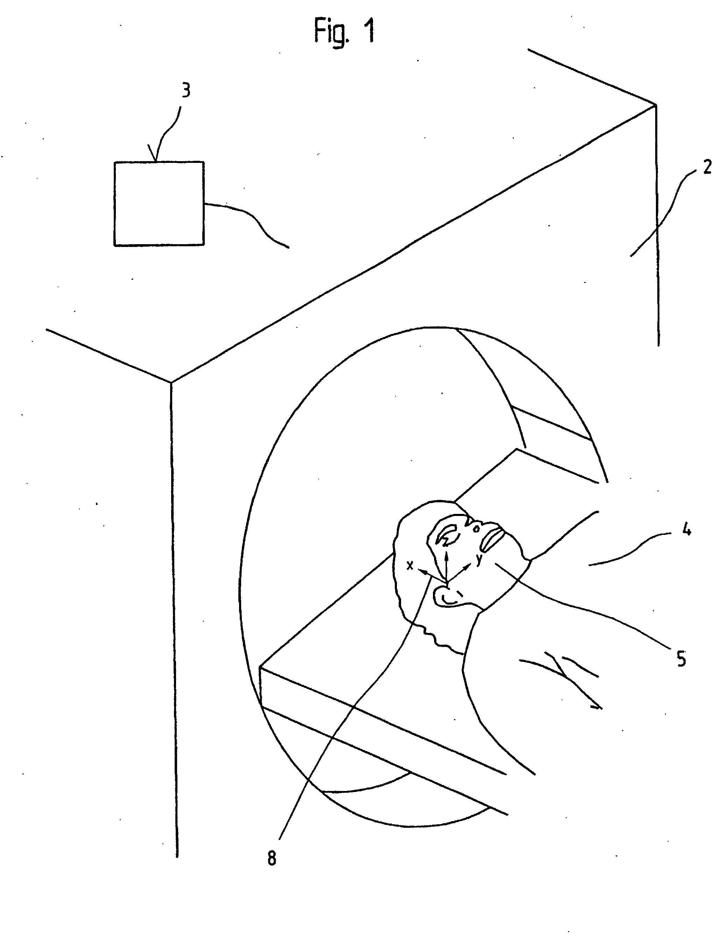

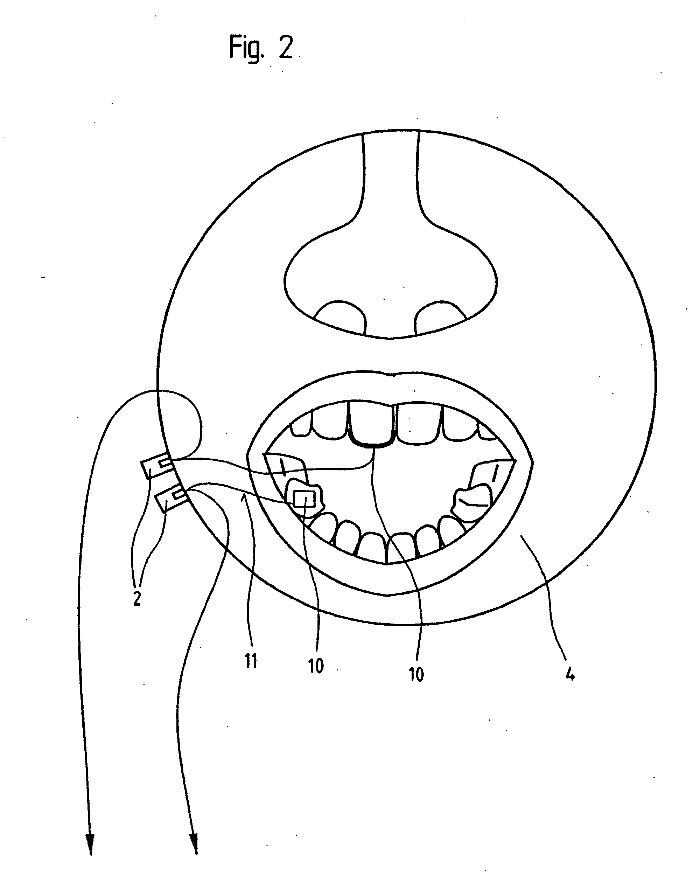

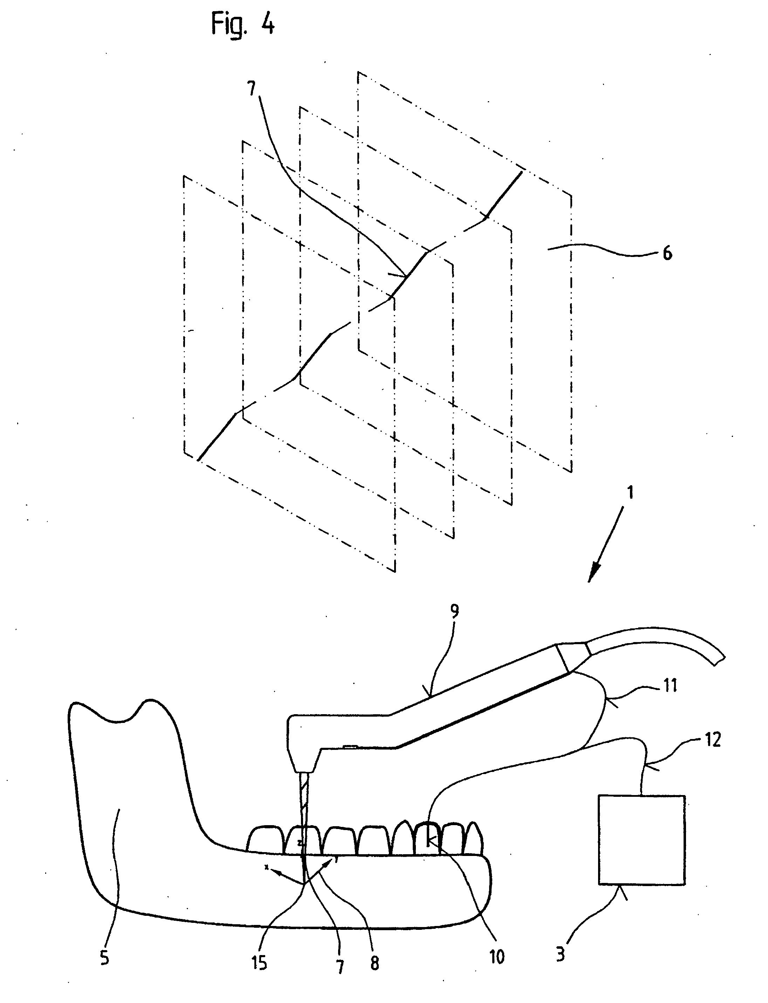

In den Figuren 1 und 2 ist ein Patient 4 in Vorbereitung auf eine dentale Operation am Ober- und/oder Unterkiefer 5 in liegender Position dargestellt. Bevor der Operateur eine dentale Operation durchführen kann, ist es erforderlich, dass die medizinischen Gegebenheiten im Ober- oder Unterkiefer 5 des Patienten 4 durch Schichtbildaufnahmen 6 ermittelt werden. Zu diesem Zweck wird der Patient 4 in eine Datenverarbeitungseinrichtung 2, beispielsweise einem Kernspintomographen, eingeführt, und der zu operierende Bereich wird mittels den Schichtbildaufnahmen 6 räumlich dargestellt. Anschließend legt der Operateur anhand der Schichtbildaufnahmen 6 einen Bohrkanal 7 fest, wie dies in der Figur 4 dargestellt ist, der den in dem Unterkiefer 5 verlaufenden Nervensträngen ausweicht und ausreichend Abstand zu den Seitenwänden des Unterkiefers 5 aufweist, so dass beispielsweise ein Implantat in den derart gebildeten Bohrkanal 7 zur Halterung eines Zahnersatzes eingesetzt werden kann.

Um die räumlichen Gegebenheiten des Patienten 4 den erstellten Schichtbildaufnahmen 6 zu zu ordnen, ist es erforderlich, dass die Schichtbildaufnahmen 6 jeweils in einem vorgegebenen am Patienten 4 ortsfest angebrachten Kartesischen Koordinatensystem 8 mathematisch eingeordnet werden können, so dass die Position des Patienten 4, insbesondere dessen Ober- oder Unterkiefer 5, in diesem Koordinatensystem 8 erfolgt. Zu diesem Zweck wird an dem Kopf des Patienten 4 mindestens ein Referenzpunkte 10 festgelegt. Die idealisierte Ausgangssitutation zur Erstellung der Schichtbildaufnahmen 6 ist die, dass der Patient 4 in einer vorgegebenen Sollposition durchleuchtet wird, so dass die Schichtbildaufnahmen 6 von dieser Sollposition nicht abweichen. Jedoch kann ein Verrutschen oder Bewegen des Kopfes des Patienten 4, beispielsweise aufgrund von Atmung oder sonstigen unbeabsichtigten Lageänderungen nicht ausgeschlossen werden, so dass mittels des vorgegebenen Referenzpunktes 10 für jede Schichtbildaufnahme 6 die tatsächliche Istposition des Patienten 4 gemessen wird. Die Abweichung der Istposition zu der angenommenen Sollposition kann demnach nachfolgend errechnet werden, so dass für jede Schichtbildaufnahme 6 eine Transformationsmatrix zur Verfügung steht, die diese unbeabsichtigte Positionsabweichung des Patienten 4 während der Erstellung der Schichtbildaufnahmen 6 berücksichtigt.

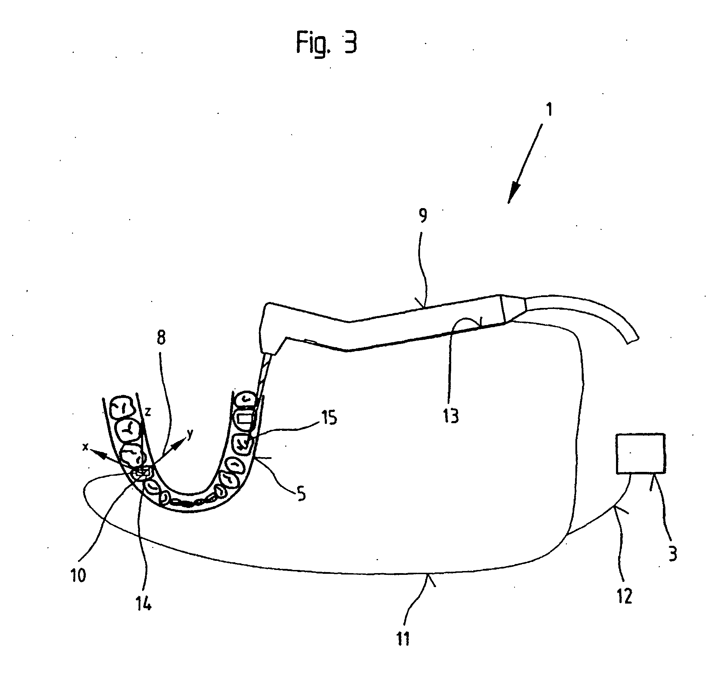

Insbesondere der Figur 2 ist zu entnehmen, dass die Datenverarbeitungseinrichtung 2 über ein Koordinatenmeßsystem 11 mit zwei Referenzpunkten 10 derart gekoppelt ist, dass die Position der Referenzpunkte 10 im Raum permanent gemessen wird und folglich eine exakte Zuordnung der jeweiligen Position zu der jeweiligen Schichtbildaufnahme 6 möglich ist. Nachdem die Schichtbildaufnahmen 6 angefertigt und der Bohrkanal 7 festgelegt ist, wird der Patient 4 in den Operationssaal gefahren. Die Referenzpunkte 10 werden - wie dies aus der Figur 3 ersichtlich ist - nicht verändert; vielmehr wird ein Ende des Koordinatenmeßsystems 11 nunmehr an ein chirurgisches Instrument 9 angeschlossen und das gegenüberliegende Ende des Koordinatenmeßsystems 11 verbleibt unverändert an dem Referenzpunkt 10, der während der Erstellung der Schichtbildaufnahmen 6 festgelegt war.

Aus Figur 3 ist ersichtlich, dass eine Vorrichtung 1 zur Durchführung der dentalen Operation am Patienten 4, insbesondere an dessen Unterkiefer 5, eingesetzt wird. Die Vorrichtung 1 umfasst dabei das chirurgisches Instrument 9, beispielsweise einen Bohrer mit einer Instrumentenspitze 15. Die Instrumentenspitze 15 soll durch den Operateur exakt entlang des festgelegten Bohrkanals 7 geführt werden. Der Operateur soll während der Operation die Möglichkeit haben, vorhandene Abweichungen der Instrumentenspitze 15 von dem vorgegebenen Bohrkanal 7 unmittelbar auf einem nicht dargestellten Monitor zu sehen und falls die Instrumentenspitze 15 vom Bohrkanal 7 abweicht, in entsprechender Weise durch Neuausrichtung und Positionierung der Instrumentenspitze 15 wieder exakt auf den vorgegebenen Bohrkanal 7 zu überführen.

Zur Überwachung der Position und Orientierung der Instrumentenspitze 15 ist an dem chirurgischen Instrument 9 das Koordinatenmeßsystem 11 angebracht. Der Befestigungspunkt des Koordinatenmeßsystems 11 am chirurgischen Instrument 9 ist mit der Bezugsziffer 13 versehen. Die Anbringung kann jedoch an jeder beliebigen Position am Instrument 9 erfolgen, da aufgrund der bekannten Instrumentengeometrie ohne weiteres ausgehend von dem Befestigungspunkt 13 der räumliche Abstand der Instrumentenspitze 15 zu dem Befestigungspunkt 13 errechnet werden kann.

Zur Bestimmung der räumlichen Position im Koordinatensystem 8 ist das Instrument 9 über das Koordinatenmeßsystem 11 mit einer Rechen- und Auswerteeinheit 3 verbunden, deren Position bezogen auf das Patienten-Koordinatensystem 8 bekannt und ortsfest ist.
Das Koordinatenmeßsystem 11 besteht aus mindestens sechs einzelnen von einer Lichtquelle durchleuchteten Glasfasersträngen, so dass aufgrund der Biegung der Glasfaserstränge in der Rechen- und Auswerteeinheit 3 die Positionswerte des Instrumentes 9 und somit der Instrumentenspitze 15 errechnet werden. Als Berechnungsgrößen dienen insbesondere die Brechung, Reflexion und Absorption des die Glasfaserstränge durchleuchtenden Lichts.
Das gegenüberliegende Ende des Koordinatenmeßsystems 11 ist weiterhin an einem Zahn des Unterkiefers 5 des Patienten 4 befestigt; dieser Befestigungspunkt am Unterkiefer 5 entspricht exakt dem während der Erstellung der Schichtbildaufnahmen 6 festgelegten Referenzpunkt 10.
Darüber hinaus kann auch jeder beliebige ortsfeste Position am Ober- oder Unterkiefer 5 des Patienten 4 als Referenzpunkt 10 ausgewählt werden, beispielsweise der Knochen des Ober- oder Unterkiefers 5, ein im Ober- oder Unterkiefer 5 befestigter Splint oder eine an dem Unterkiefer 5 fixierte Schablone, die mit dem Unterkiefer 5 verklemmt ist.

Um die im Koordinatenmeßsystem 11 vorhandenen Positionssignale auswerten zu können, ist das Koordinatenmeßsystem 11 über eine elektrische Leitung 12 mit der Rechen- und Auswerteeinheit 3 verbunden.
Aufgrund dieser Anordnung der Vorrichtung 1 ist es möglich, dass die Position der Instrumentenspitze 15 mit der vorher festgelegten Position des Bohrkanals 7 in Abhängigkeit der Bohrungstiefe exakt in einem Koordinatensystem 8 abgelichen werden kann..
Dies ist insbesondere aus Figur 4 zu entnehmen, in der zum einen die angefertigten Schichtbildaufnahmen 6 mit dem festgelegten Bohrkanal 7 und zum anderen der tatsächliche Bohrkanal 7 im Unterkiefer 5 des Patienten 4 dargestellt sind. Für jede Bohrungstiefe ist eine Schichtbildaufnahme 6 vorgesehen, die optisch dem Operateur vorgeführt wird. Aufgrund der ermittelten Messergebnisse der Positionswerte der Instrumentenspitze 15 kann somit eine Abweichung der Instrumentenspitze 15 von dem vorgegebenen Bohrkanal 7 anhand der Schichtbildaufnahmen 6 unverzüglich und exakt auf dem Monitor dargestellt werden und der Operateur kann gegebenenfalls die Instrumentenspitze 15 in dessen Lage und Orientierung verändern. Falls die Instrumentenspitze 15 von dem vorgegebenen Bohrkanal 7 abweichen sollte, erkennt der Operateur, in welche Richtung und unter welchem Winkel die Instrumentenspitze 15 nachzuführen ist, damit die Instrumentenspitze 15 wieder entlang des vorgegebenen Bohrkanals 7 positioniert ist.

## Patentansprüche

1. Verfahren zur Steuerung eines Systems zur Bestimmung und Abgleichung der Raumkoordinaten eines chirurgischen Instrumentes (9) während einer dentalen Operation am Ober- und/oder Unterkiefer (5) eines Patienten (4), wobei an dem-zu operierenden Kiefer (5) mittels einer Datenverarbeitungseinrichtung (2) Schichtbildaufnahmen (6) erstellt und dabei mindestens ein dem Ober- und/ oder Unterkiefer (5) zugeordneter Referenzpunkt (10) festgelegt wird, dessen Raumkoordinaten, bezogen auf ein kartesisches Koordinatensystem (8), bestimmt und dem jeweiligen Schichtbild (6) zugewiesen werden, dass während der anschließenden dentalen Operation die Raumkoordinaten des chirurgischen Instrumentes (9) mit Hilfe eines an dieses und an eine Rechen- und Auswerteeinheit (3) angeschlossenen Koordinatenmeßsystems (11) in dem festgelegten Koordinatensystem (8) ermittelt werden und wobei sodann die ermittelten Raumkoordinaten des Instrumentes (9) mit Hilfe der Rechen- und Auswerteeinheit (3) mit den Raumkoordinaten einer vorgegebenen auf den Schichtbildern (6) dargestellten Operationsposition abgeglichen und an diese durch Nachführen des Instrumentes (9) angepasst werden,
**dadurch gekennzeichnet,**
**dass** die Zuordnung eines oder mehrerer Referenzpunkte (10) zu dem jeweiligen Schichtbild (6) und die Messung der Raumkoordinaten des Instrumentes (9) dadurch erfolgen, dass das Koordinatenmeßsystem (11) zunächst an einem oder an mehreren Referenzpunkten (10) und der Datenverarbeitungseinrichtung (2) und anschließend an dem einen oder an mehreren Referenzpunkten (10) und einem Ende des Instrumentes (9) angebracht ist und dass das Koordinatenmeßsystem (11) über eine elektrische Leitung (12) mit der Rechen- und Auswerteeinheit (3) verbunden ist.

2. Verfahren nach Anspruch 1,
**dadurch gekennzeichnet,**
**dass** während der Erstellung der Schichtbildaufnahmen (6) des zu operierenden Kiefers (5) die Positionsabweichung des Kiefers (5) für jede Schichtbildaufnahme (6) an Hand der Positionsbestimmung der Referenzpunkte (10) ermittelt wird.

3. Verfahren nach Anspruch 2,
**dadurch gekennzeichnet,**
**dass** durch die ermittelten Positionsabweichungen der Referenzpunkte (10) eine Transformationsmatrix errechnet wird, mittels der die Schichtbildaufnahmen (6) in Abhängigkeit von einem vorgegebenen Istwert kalibriert werden.

4. Verfahren nach Anspruch 3,
**dadurch gekennzeichnet,**
**dass** durch die Transformationsmatrix die Positionsabweichung des zu operierenden Ober- oder Unterkiefers (5) bezogen auf den vorgegebenen Istwert ermittelt wird.

5. Verfahren nach einem oder mehreren der vorgenannten Ansprüche,
**dadurch gekennzeichnet,**
**dass** der Ursprung des kartesischen Koordinatensystems (8), in dem die Raumkoordinaten in Position und Orientierung des chirurgischen Instrumentes (9) erfasst werden, einem Referenzpunkt (10) entspricht.

6. Verfahren nach Anspruch 5,
**dadurch gekennzeichnet,**
**dass** das Koordinatenmeßsystem (11) unmittelbar an einem Referenzpunkt (10), vorzugsweise an einem Zahn des Ober- oder Unterkiefers (5), an einem Knochen des Ober- oder Unterkiefers (5), an einem im Ober- oder Unterkiefer (5) angeordneten Splint oder an einer mit dem Ober- oder Unterkiefer (5) verklemmten Schablone, befestigt ist und dass das Koordinatenmeßsystem (11) permanent an dem Referenzpunkt (10) unverändert in seiner Position bis zum Abschluss der dentalen Operation befestigt ist

7. Vorrichtung (1) zur Anwendung des Verfahrens nach einem oder mehreren der Ansprüche 1 bis 7, mit einer Datenverarbeitungseinrichtung (2) zur optischen Darstellung von dreidimensionalen Schichtbildaufnahmen (6) des zu operierenden Ober- oder Unterkiefers (5) sowie mit einer Rechen- und Auswerteeinheit (3) zur Bestimmung der örtlichen Position des Instrumentes (9) während der Operation,
**dadurch gekennzeichnet,**
**dass** das Instrument (9) mit der Rechen- und Auswerteeinheit (3) über mindestens ein Koordinatenmeßsystem (11) verbunden ist und dass die Datenverarbeitungseinrichtung (2) und mindestens ein Referenzpunkt (10) am Oberund/ oder Unterkiefer (5) des Patienten (4) während der Erstellung der Schichtbildaufnahmen (6) mit dem Koordinatenmeßsystem (11) sowie der Rechenund Auswerteeinheit (3) gekoppelt sind.

8. Vorrichtung nach Anspruch 7,
**dadurch gekennzeichnet,**
**dass** das Koordinatenmeßsystem (11) aus mindestens sechs einzelnen mit Lichtstrahlen durchleuchteten Glasfasersträngen besteht und dass die Biegung, Reflexion, Brechung und Absorption der Glasfaserstränge als Berechnungsgrößen dienen, mittels denen die Rechen- und Auswerteeinheit (3) die Raumkoordinaten des am Instrument (9) befestigten Endes der Glasfaserstränge errechnet.

9. Vorrichtung nach Anspruch 7 oder 8,
**dadurch gekennzeichnet,**
**dass** durch die Rechen- und Auswerteeinheit (3) jeder erstellten Schichtbildaufnahme (6) ein Abweichungsparameter zugeordnet ist, mittels dem eine Transformationsmatrix in einem einheitlichen Kartesischen Koordinatensystem (8) erstellbar ist.

## Claims

1. A method for controlling a system for determination and calibration of coordinates of a surgical instrument (9) during a dental operation on the upper and/or lower jaw (5) of a patient (4), in which tomographs (6) are produced by means of a data processing device (2), in the process of which at least one reference point (10) for the upper and/or lower jaw (5) is defined, the coordinates of the aforementioned reference point (10) are determined in relation to a Cartesian system of coordinates (8) and are assigned to the particular tomograph (6), in which, during the subsequent dental operation, the coordinates of the surgical instrument (9) are ascertained within the defined system of coordinates (8) by means of a coordinate measuring system (11) connected to the surgical instrument (9) and to a computation and evaluation unit (3) and in which the ascertained coordinates of the instrument (9) are then calibrated by means of the computation and evaluation unit (3) in relation to the operation position shown on the tomographs (6) and are adapted to this position by adjusting the position of the instrument (9),
**characterised in that**,
one or more reference points (10) are assigned to the particular tomograph (6) and the coordinates of the instrument (9) are measured by connecting the coordinate measuring system (11) to one or more reference points (10) and to the data processing device (2) and subsequently connecting the coordinate measuring system (11) to the one reference point (10) or several reference points (10) and to one end of the instrument (9), and that the coordinate measuring system (11) is connected to the computation and evaluation unit (3) by means of an electrical cable (12).

2. The method in accordance with Claim 1,
**characterised in that**,
while the tomographs (6) are being taken of the jaw (5) to be operated on, the positional deviation of the jaw (5) is ascertained for each tomograph (6) by means of the position determination of the reference points (10).

3. The method in accordance with Claim 2,
**characterised in that**,
the ascertained positional deviations of the reference points (10) are used to calculate a transformation matrix by means of which the tomograms (6) can be calibrated in relation to a specified actual value.

4. The method in accordance with Claim 3,
**characterised in that**,
the positional deviation of the upper or lower jaw (5) to be operated on is ascertained in relation to the specified actual value by means of the transformation matrix.

5. The method in accordance with one or more of the aforementioned claims,
**characterised in that**,
the origin of the Cartesian system of coordinates (8) within which the positional and orientation coordinates of the surgical instrument (9) are registered corresponds to a reference point (10).

6. The method in accordance with Claim 5,
**characterised in that**,
the coordinate measuring system (11) is directly attached to a reference point (10), preferably to a tooth of the upper or lower jaw (5), to a bone of the upper or lower jaw (5), to a pin arranged in the upper or lower jaw (5) or to a template clamped onto the upper or lower jaw (5), and that the coordinate measuring system (11) remains permanently attached to the reference point (10) without changing its position until the completion of the dental operation.

7. A device (1) for using the method in accordance with one or more of Claims 1 to 7, with a data processing device (2) for visually displaying three-dimensional tomographs (6) of the upper or lower jaw (5) to be operated on and with a computation and evaluation unit (3) for determining the spatial position of the instrument (9) during the operation,
**characterised in that**,
the instrument (9) is connected to the computation and evaluation unit (3) by means of at least one coordinate measuring system (11), and that the data processing device (2) and at least one reference point (10) on the upper and/or lower jaw (5) of the patient (4) are connected to the coordinate measuring system (11) and to the computation and evaluation unit (3) whilst the tomographs (6) are being taken.

8. The device in accordance with Claim 7,
**characterised in that**,
the coordinate measuring system (11) comprises at least six individual glass fibres through which beams of light shine, and that the bending, reflection, refraction and absorption of the glass fibres serve as calculation parameters by means of which the computation and evaluation unit (3) calculates the coordinates of the end of the glass fibres connected to the instrument (9).

9. The device in accordance with Claim 7 or 8,
**characterised in that**,
a deviation parameter is assigned to each tomograph (6) by the computation and evaluation unit (3), thereby allowing a transformation matrix to be created within a uniform Cartesian system of coordinates (8).

## Revendications

1. Procédé de commande d'un système servant à déterminer et à équilibrer les coordonnées spatiales d'un instrument chirurgical (9) pendant une opération dentale sur la mâchoire supérieure et/ou la mâchoire inférieure (5) d'un patient (4), où il est fait des tomographies (6) au moyen d'un ordinateur (2) sur la mâchoire à opérer (5), où il est fixé au moins un point de référence (10) attribué à la mâchoire supérieure et/ou la mâchoire inférieure (5), point dont les coordonnées spatiales sont déterminées avec référence à un système de coordonnées cartésiennes (8) et attribuées à la tomographie respective (6), que lors de l'opération dentale suivante, les coordonnées spatiales de l'instrument chirurgical (9) sont déterminées dans le système de coordonnées (8) fixé à l'aide d'un système de mesure des coordonnées (11) raccordé à l'instrument ainsi qu'à une unité de calcul et d'évaluation (3) et où les coordonnées spatiales calculées de l'instrument (9) sont ensuite comparées à l'aide de l'unité de calcul et d'évaluation (3) avec les coordonnées spatiales d'une position d'opération réglée et représentée sur les tomographies (6), et ajustées sur celles-ci par réglage de l'instrument (9),
**caractérisé en ce que**
l'attribution d'un ou de plusieurs points de référence (10) à la tomographie respective (6) et la mesure des coordonnées spatiales de l'instrument (9) sont effectuées de façon à ce que le système de mesure des coordonnées (11) soit appliqué d'abord sur un ou sur plusieurs points de référence (10) et sur l'ordinateur (2) et ensuite sur l'un ou sur plusieurs points de référence (10) et sur une extrémité de l'instrument (9), et que le système de mesure des coordonnées (11) est raccordé par une ligne électrique (12) avec l'unité de calcul et d'évaluation (3).

2. Procédé d'après la revendication 1,
**caractérisé en ce que**
pendant la préparation des tomographies (6) de la mâchoire à opérer (5), le déplacement de la mâchoire (5) est déterminé pour chaque tomographie (6) à l'aide de la détermination de la position des points de référence (10).

3. Procédé d'après la revendication 2,
**caractérisé en ce que**
à l'aide des déplacements déterminés des points de référence (10), il est établi une matrice de transformation servant au calibrage des tomographies (6) en dépendance d'une valeur effective prédéterminée.

4. Procédé d'après la revendication 3,
**caractérisé en ce que**
moyennant la matrice de transformation, il est déterminé le déplacement de la mâchoire supérieure ou inférieure à opérer (5) en dépendance d'une valeur effective prédéterminée.

5. Procédé d'après une ou plusieurs des revendications précédentes,
**caractérisé en ce que**
l'origine du système de coordonnées cartésiennes (8), dans lequel sont saisies les coordonnées spatiales pour la position et l'orientation de l'instrument chirurgical (9), correspond à un point de référence (10).

6. Procédé d'après la revendication 5,
**caractérisé en ce que**
le système de mesure des coordonnées (11) est raccordé directement sur à un point de référence (10), de préférence une dent de la mâchoire supérieure ou inférieure (5), à un os de la mâchoire supérieure ou inférieure (5), à une goupille prévue dans la mâchoire supérieure ou inférieure (5) ou à un gabarit coincé sur la mâchoire supérieure ou inférieure (5), et que, jusqu'à la fin de l'opération dentale, le système de mesure des coordonnées (11) est fixé en position invariable et en permanence sur le point de référence (10).

7. Dispositif (1) pour l'application d'un procédé d'après une ou plusieurs des revendications 1 à 6, avec un ordinateur (2) servant à la représentation optique des tomographies tridimensionnelles (6) de la mâchoire supérieure ou inférieure à opérer (5), ainsi qu'avec une unité de calcul et d'évaluation (3) servant à la détermination de la position locale de l'instrument (9) pendant l'opération,
**caractérisé en ce que**
l'instrument (9) est raccordé par l'intermédiaire d'au moins un système de mesure des coordonnées (11) avec l'unité de calcul et d'évaluation (3) et que pendant la préparation des tomographies (6), l'ordinateur (2) et au moins un point de référence (10) sur la mâchoire supérieure et/ou la mâchoire inférieure (5) du patient (4) sont liés au système de mesure des coordonnées (11) ainsi qu'à l'unité de calcul et d'évaluation (3).

8. Dispositif d'après la revendication 7,
**caractérisé en ce que**
le système de mesure des coordonnées (11 ) consiste en au moins six fils de fibres de verre individuels traversés par de la lumière et que la flexion, la réflexion, la réfraction et l'absorption des fils de fibres de verre servent de paramètres de calcul au moyen desquels l'unité de calcul et d'évaluation (3) détermine les coordonnées spatiales de l'extrémité des fils de fibres de verre fixée sur l'instrument (9).

9. Dispositif d'après la revendication 7 ou 8,
**caractérisé en ce que**
l'unité de calcul et d'évaluation (3) attribue à chaque tomographie (6) un paramètre d'écartement permettant d'établir une matrice de transformation dans un système de coordonnées cartésiennes unique (8).
